# EUROPEAN PATENT APPLICATION

(11) **EP 1 935 414 A2**
(43) Date of publication of application: **25.06.2008**
(21) Application number: 08003718.7
(22) Date of filing: 02.05.2002
(51) Int. Cl.: A61K 31/137, A61K 31/198, A61K 31/4164, A61K 31/4178, A61K 31/417, A61K 31/4172, A61P 25/28

(54) **Carbonic anhydrase activators for enhancig learning and memory**

(30) Priority: 02.05.2001 US 287721 P
(62) Divisional of application: 02725885.4
(71) Applicant: Blanchette Rockefeller Neurosciences Institute, Rockville, MD 20850 (US)
(72) Inventor: Alkon, Daniel, Bethesda, MD 20817 (US); Sun, Miao-Kun, Bethesda, MD 20878 (US)
(74) Representative: Kador & Partner

(57) **Abstract**

The invention provides activator compounds for improving attentive cognition comprising administering a compound that potentiates intraneuronal carbonic anhydrase activity thereby improving establishment of a theta rhythm.

## Description

This application claims the benefit of provisional application USSN 60/287,721, filed May 2, 2001, incorporated herein by reference.

### BACKGROUND OF THE IIVVENTION

This invention relates to methods and compositions for improving attention, learning, and memory by activating carbonic anhydrase. Drugs that enhance acquisition and/or recall of associative memory represent important goals in the therapy of cognitive disorders. The effectiveness of such therapy depends on whether the targeted mechanisms are actually involved in memory itself. Learning and memory are believed to require modifications of synaptic strength among relevant neurons in the network, through an interaction of multiple afferent pathways and signal molecules (Christie et al., 1994; Kornhauser and Greenberg, 1997; Ohno et al., 1997; Alkon et al., 1998; Paulsen and Moser, 1998; Xiang et al., 1998 Tang et al., 1999; Wu et al., 2000). A requirement for multiple synaptic interactions, versus a single glutamatergic pathway often studied experimentally, is in fact consistent with characterization of multiple deficits of neurotransmitters in memory impairments, including Alzheimer's disease. Targeting the relevant synaptic/signal interactions within memory traces therefore might be an effective way to achieve a specific effect on learning and memory pharmacologically.

In mammals, the essential role of hippocampal CA1 pyramidal cells in spatial memory is well established. The CA1 pyramidal cells receive, in addition to glutamatergic input from the CA3 pyramidal neurons, abundant cholinergic and GABAergic inputs. Activation of the medical septal afferents within the perforant pathway, a major cholinergic input to the hippocampus (Cooper and Sofroniew, 1996), is believed to be required for associative learning (Dickinson-Anson et al., 1998; Perry et al., 1999), since its disruption abolishes spatial memory (Winson, 1978; Winkler et al., 1995). GABAergic interneurons, on the other hand, control hippocampal network activity and synchronize the firing of pyramidal cells (Buhl et al., 1995; Cobb et al., 1995; Banks et al., 2000). One GABAergic interneuron is known to innervate some 1000 pyramidal cells, effectively shutting down the signal outflow when the interneurons are active (Sun et al., 2000). The functional interaction between these major inputs thus plays a significant role in hippocampus-dependent memory (Bartus et al., 1982; Winkler et al., 1995; Paulsen and Moser, 1998) and has attracted much attention in an effort to "dissect" the memory traces.

Consistent with the observations that the GABAergic synaptic responses can be switched from inhibitory to excitatory (Alkon et al., 1992; Collin et al., 1995; Kaila et al., 1997; Taira et al., 1997; Sun et al., 2000, 2001b), evidence has been provided that such a synaptic switch depends on the increased HCO₃ conductance through the GABA_{A} receptor-channel complex and dramatically alters the operation of signal transfer through the hippocampal network (Sun et al., 1999, 2000). The synaptic switch appears to depend on carbonic anhydrase, a zinc-containing enzyme that catalyzes the reversible hydration of carbon dioxide. Carbonic anhydrase is present within the intracellular compartments of the pyramidal cells (Pastemack et al., 1993). The fact that a membrane-impermeant carbonic anhydrase inhibitor, benzol amide, was effective in blocking the synaptic switch when introduced into the recorded pyramidal cells, but not when applied extracellularly (Sun et al., 1999), indicates that the underlying enzyme is intracellular. Blocking the rapid HCO₃ formation that depends on carbonic anhydrase activity thus prevents the synaptic switch in vitro and impairs rat spatic plasticity and memory.

Acetazolamide, a known inhibitor of carbonic anhydrase activity, inhibits theta rhythm, learning, and memory. Sun MK, Zhao WQ, Nelson TJ, Alkon DL., "Theta Rhythm of Hippocampal CA1 Neuron Activity: Gating by GABAergic Synaptic Depolarization," J Neurophysiol 2001 Jan;85(1):269-79. Prior data showing that inhibition of carbonic anhydrase activity impaired memory formation was not predictive that activation would enhance memory formation. For example, it was not known if the enzyme was already operating at a maximal level in neurons involved with learning, which could not be further activated. It was also not known if there are homeostatic mechanisms in such cells that would neutralize any activation due to administration of a compound according to the invention.

Pending patent application PCT/US01/18329, filed June 7, 2001 by the National Institutes of Health, incorporated herein by reference, disclosed that activating carbonic anhydrase can lead to improved learning and memory. There is a long-felt need for compounds and pharmaceutical agents that activate carbonic anhydrase and improve learning and memory in mammals.

### SUMMARY OF THE INVENTION

The invention provides methods for improving attention and/or memory acquisition comprising stimulating intraneuronal carbonic anhydrase activity. The stimulation is achieved by administering a carbonic anhydrase activator. The method allows treating neurodegenerative disorders to enhance cognitive ability, treating dementia, and also enhancing attention and learning in healthy individuals.

The invention provides a method for improving attentive cognition comprising administering a compound that potentiates intraneuronal carbonic anhydrase activity thereby improving establishment of a theta rhythm.

The invention provides a method comprising administering to the brain of a subj ect in need of improved attentive cognition a carbonic anhydrase activator compound in a dose effective to improve attentive cognition, the carbonic anhydrase activator compound being selected from the groups of structure I, II, or III described below. The compound may potentiate intraneuronal carbonic anhydrase activity. The compound may be structure I wherein R¹ is H or OH; R² is H, CH₃ or COOH; R³ is H or CH₃; and Ar is H, phenyl, 4-hydroxyphenyl, 4-fluorophenyl, 4-aminophenyl, 3-amino-4-hydroxyphenyl, 3,4-dihydroxyphenyl, imidazole, imadazol-4-yl-, or 5-methylimidazole-4-yl-. The activator may have structure II wherein R¹ is H, methyl or ethyl; and R² is H or methyl. The activator may have structure III wherein *n* is 1 or 2; and R² is H or methyl. The activator may be imidazole, alanine, phenylalanine, substituted ethylamine, phenethylamine, histamine, histidine, linked di-imidazole, triazole, and/or salts thereof.

The carbonic anhydrase activator may be administered as a pharmaceutical composition or in a pharmaceutically acceptable carrier, or as a prodrug that metabolizes to form a compound of the invention and deliver that drug to the brain of a subject.

The patient may have a neurodegenerative disorder or the method enhances cognitive ability, attention, learning, and/or memory in individuals without a neurological disorder.

The method may facilitate establishment of a theta rhythm via bicarbonate-mediated GABAergic depolarization. The method may improve memory formation, learning, spatial memory, and/or attention. The method may intervene in the intracellular signaling cascade responsible for theta rhythm, the intervention comprising modulating HCO₃⁻ conductance by directly altering intraneuronal carbonic anhydrase activity. The intervention may modulate the HCO₃ current relative to the Cl⁻ and K⁺ currents.

The method may improve attentive cognition in a subject with Alzheimer's disease, stroke, hypoxia, and/or ischemia.

The method may employ a compound that provides carbonic anhydrase activity at least about 150%, 200%, or 250% that of alanine in vitro.

The invention relates to an article of manufacture comprising a pharmaceutical composition comprising an activator compound or prodrug thereof packaged together with labeling indicating use for improving attentive cognition, the activator compound being effective to enhance brain carbonic anhydrase activity and selected from structures I, II, or III, or salts thereof.

Further objectives and advantages will become apparent from a consideration of the description, drawings, and examples.

### BRIEF DESCRIPTION OF THE DRAWINGS

The invention is better understood by reading the following detailed description with reference to the accompanying figures:

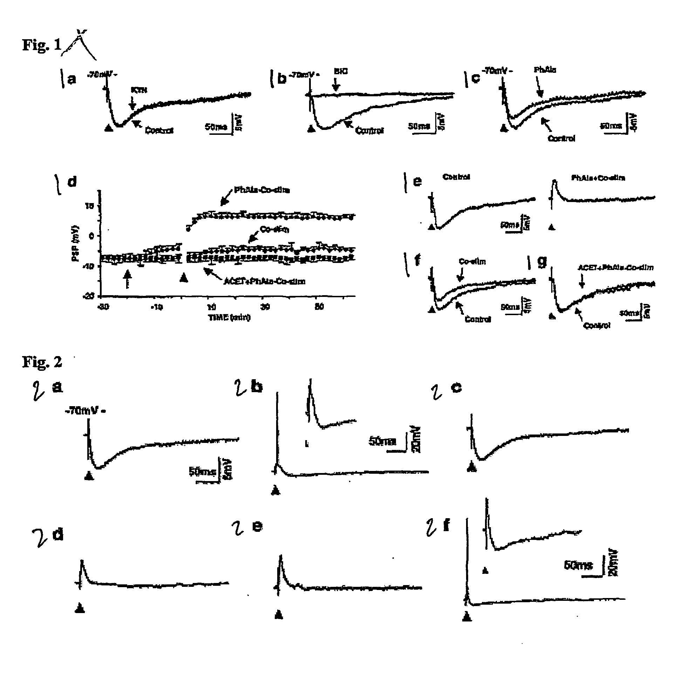

Figures 1a, 1b, 1c, 1d, 1e, 1f and 1g demonstrate the associated activation of cholinergic and GABAergic inputs and carbonic anhydrase induced long-term synaptic switching from an inhibitory to excitatory response. Single-pulse stimulation of stratum pyramidale (50 µA, 50 µs) evokes an IPSP (control), which is not changed by bath kynurenic acid (KYN; 500 µM, 20 min; Fig. 1a). The IPSP (control), however is eliminated by bicuculline (BIC; 1 µM, 30 min; Fig. 1b). The application of phenylalanine (100 µM, starting at the vertical arrow in d) reduces the IPSP slightly when applied alone (Fig. 1c) but induces a lasting synaptic reversal of the GABAergic responses when association with costimulation (at the arrowhead in Fig. 1d; under *Materials and Methods)* of stratum oriens and stratum pyramidale (PhAla + Co-stim; Fig. 1d and Fig. 1e). The same costimulation, however, does not trigger the synaptic switch (Co-stim; Fig. 1d and Fig. 1f) and the effects of phenylalaline-costimulation on the synaptic switch are eliminated (ACET + PhAla-Co-stim; Fig. 1d and Fig. 1g) by the application of acetazolamide (10 µM, also starting at the vertical arrow in Fig. 1d). Arrowheads indicate the time when single-pulse stimulation of stratum pyramidale is delivered In d, the data points are illustrated as means ± standard errors of the means and for clarity, only every other minute is illustrated.
Figures 2a, 2b, 2c, 2d, 2e and 2f shows how synaptic switch converts excitatory input filter into amplifier. Single-pulse stimulation of stratum pyramidale evokes an IPSP (Fig. 2a). Single pulse stimulation of Sch at above-threshold intensity evokes an action potential (Fig. 2b). Co-single-pulse stimulation of stratum pyramidale and Sch (the same as Fig. 2a and Fig. 2b) eliminates the EPSP and no action potential is evoked (Fig. 2c). After the associated costimulation of stratum pyramidale and stratum oriens (under *Materials and Methods)* in the presence of phenlyalanine, the IPSP is reversed to EPSP, observed at the same resting membrane potential (Fig. 2d). Single-pulse stimulation of Sch at below-threshold intensities evokes an EPSP (Fig. 2e). Cosingle-pulse stimulation of stratum pyramidale and Sch (the same as Fig. 2d and Fig. 2e) evokes an action potential (Fig. 2f). Arrowheads indicate the time when single-pulse stimulation of stratum pyramidale or costimulation is delivered. The calibration bar units are the same for the traces and insets (as in Fig. 2a) except Fig. 2b and Fig. 2f.

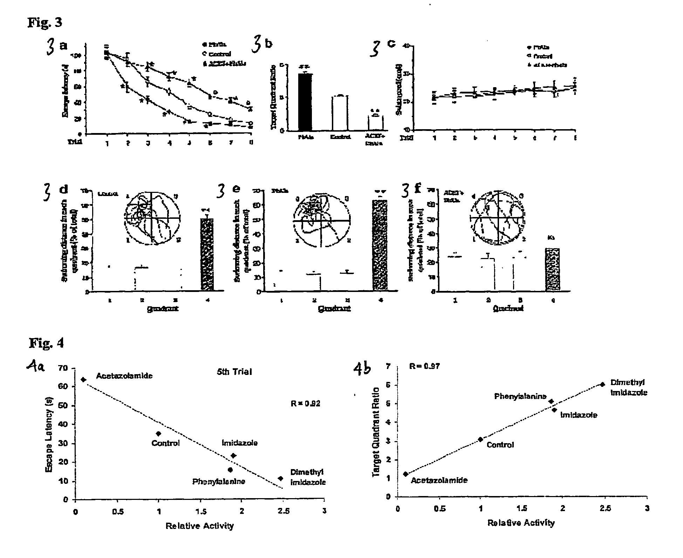

Figures 3a, 3b, 3c, 3d, 3e and 3f demonstrate how the carbonic anhydrase activator enhances rat performance in the hidden platform water maze task. The figure illustrates escape latency (means ± standard errors of the means; n = 10 for each group) in water maze training (Fig. 3a) across eight trials (*F*_{7,105} = 55.78, *p* < 0.0001), swim speeds (Fig. 3c), and quadrant preference (Fig. 3d-f) conducted at the end of the eighth training session. Quadrant 4 is the target quadrant during training. Insets are paths taken by representative rats with quadrant numbers indicated. The target ratio is defined as the time searching in the target quadrant/the average of the nontarget quadrants (Fig. 3b).
Figures 4a and 4b show a linear correlation between the relative activity of carbonic anhydrase in the presence of the activator compound and the escape latency (Fig. 4a), which reflects learning, and the target quadrant ratio (Fig. 4b) which reflects memory. Techniques were as described in the Examples.

### DETAILED DESCRIPTION OF THE PREFERRED EMBODIMENTS

In describing preferred embodiments of the present invention illustrated in the drawings, specific terminology is employed for the sake of clarity. However, the invention is not intended to be limited to the specific terminology so selected. It is to be understood that each specific element includes all technical equivalents which operate in a similar manner to accomplish a similar purpose. Each reference cited here is incorporated by reference as if each were individually incorporated by reference.

According to the invention, one can administer a drug to a patient at a given time to produce a cognitive effect (referred to as attentive cognition), such as learning, learning-related attention, associative learning, and memory acquisition, and memory consolidation (without affecting memory storage and recall) by activating neuronal carbonic anhydrase e.g. by compounds that enhance carbonic anhydrase activity and thereby switch GABAergic activity from predominantly hyperpolarizing Cl- conductance to a depolarizing, primarily HCO₃ conductance, entraining pyramidal cells into a theta rhythm.

Principal aspects of the invention include (1) specific cognitive effects, (2) theta rhythm effects, and in particular, (3) the method of enhancing learning by stimulating carbonic anhydrase activity above standard control levels. The fact that carbonic anhydrase is a common link between stimulating excitatory post synaptic potential and stimulating theta rhythm allows therapies for neurological disorders, including cognitive therapy.

The invention provides a method for improving attentive cognition comprising administering a compound that enhances intraneuronal carbonic anhydrase activity thereby affecting establishment of a theta rhythm. The metabolic pathway of the compound preferably involves bicarbonate-mediated GABAergic depolarization. The term "attentive cognition" is meant to encompass memory formation, learning, spatial memory, and attention. Attentive cognition can include one or more of attention, learning, and/or memory acquisition and/or retention, According to the invention, theta rhythm can be enhanced by carbonic anhydrase activators to treat neurological disorders such as stroke, hypoxia, and ischemia.

Administering a compound of the invention to the brain means either administering the compound itself, which crosses the blood brain barrier in an effective amount, or administering a pro-drug that is metabolized to the compound of the invention either before entering the brain or in the brain, to deliver such compounds to the brain.

Methods of measuring carbonic anhydrase activity and attentive cognition in rats have previously been published. Sun MK, Zhao WQ, Nelson TJ, Alkon DL., "Theta Rhythm of Hippocampal CA1 Neuron Activity: Gating by GABAergic Synaptic Depolarization," J Neurophysiol 2001 Jan;85(1):269-79.

The invention encompasses methods and compounds described in Sun MK, Alkon DL., "Pharmacological Enhancement of Synaptic Efficacy, Spatial Learning, and Memory Through Carbonic anhydrase Activation in Rats," J. Pharmacol. and Experimental Therapeutics 297(3):961-967 and incorporated herein by reference. In the presence of carbonic anhydrase activators, co-microstimulation of cholinergic inputs from stratum oriens and gamma-aminobutyric acid (GABA)ergic inputs from stratum pyramidale at low intensities switched hyperpolarizing GABA-mediated inhibitory postsynaptic potentials to depolarizing responses. The carbonic anhydrase activators caused rats to exhibit superior learning of the Morris water maze task, suggesting that the GABAergic synaptic switch is critical for gating the synaptic plasticity that underlies spatial memory formation. Increased carbonic anhydrase activity enhances perception, processing and storing of temporally associated relevant signals and represents an important therapy for learning and memory pharmacology.

The carbonic anhydrase activators according to the invention include, for example, imidazole, phenylalanine, and their structural analogs, derivatives and salts, as shown further by the exemplary embodiments described below. Tables 1, 2 and 3 show exemplary compounds of the invention. The activities of these compounds relative to the control level of activity for the CA-II isozyme are also presented.

Suitable activator compounds and methods for measuring carbonic anhydrase activity can be found in Clare, B.W. and Supuran, C.T., "Carbonic anhydrase activators: 3: Structure-activity correlations for a series of isozyme II activators", J. Pharmaceut. Sci. 83: 768-773, 1994; Supuran, C.T., et al., "Carbonic anhydrase activators. Part 7. Isozyme II activation with bisazolylmethanes, -ethanes and related azoles.," Biol. Pharm. Bull. 16: 1236-1239, 1993; and Supuran, C.T., et al., "Carbonic anhydrase activators: XV. A kinetic study of the interaction of bovine isozyme II with pyrazoles, bis- and tris- azolyl-methanes.," Biol. Pharm. Bull. 19: 1417-1422,1996. These references are incorporated herein by reference.

An exemplary embodiment of the present invention encompasses activator compounds generally described as having the structure: 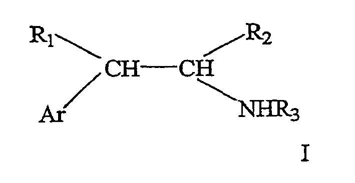 wherein R¹ is H or OH; R² and R³ are independently H, COOH or lower alkyl, for example linear, branched or cyclic C₁-C₆ alkyl or C₁-C₄ alkyl; and Ar is phenyl, imidazolyl or phenyl or imidazolyl substituted with one or more halo, hydroxy, amino or lower alkyl for example linear, branched or cyclic C₁-C₆ alkyl or C₁-C₄ alkyl. An example of an alkyl group for R² and R³ is methyl. Examples of Ar include phenyl, 4-hydroxyphenyl, 4-fluorophenyl, 4-aminophenyl, 3-amino-4-hydroxyphenyl, 3,4-dihydroxyphenyl, imidazole, imadazol-4-yl-, or 5-methylimidazole-4-yl-. Particular examples are provided in Table 1. These compounds include substituted ethylamines, including phenethylamines substituted on the aromatic or aliphatic portion. Alanine is defined as having a 100% activity as control. Phenylalanine, histamine, histidine, and other alanine derivatives are also listed in Table 1 (compounds 1-17).

**Table 1**

| **Effector** | **Ar** | **R1** | **R2** | **R3** | **% Activity/control CAII activity** | **Comments** |
|---|---|---|---|---|---|---|
| 1 | H | H | COOH | H | 100 | Alanine, ineffective at 0.2 mM |
| 2 | Phenyl | H | COOH | H | 186.7 | Phenylalanine |
| 3 | Phenyl | H | H | H | 109.5 | COOH is more effective |
| 4 | 4-Hydroxyphenyl | H | COOH | H | 189.1 | 4-hydroxy, no improvement over phenylalanine |
| 5 | 4-Fluorophenyl | H | COOH | H | 167.7 | 4-Fluoro, less effective [4-fluorophenylalanine] |
| 6 | 4-Arriinophenyl | H | COOH | H | 159.4 | 4-amino, less effective [4-aminophenylalanine] |
| 7 | 3-Amino-4-hydroxyphenyl | H | COOH | H | 176.3 | 3-amino, less effective [3-amino-4-hydroxyphenylalanine] |
| 8 | 3,4-Dihydroxyphenyl | H | COOH | H | 134.3 | Less effective with 2-OH |
| 9 | 3,4-Dihydroxyphenyl | H | H | H | 137.5 | Less effective 2-OH [2-(3,4-dihydroxyphenyl) ethanamine] |
| 10 | 3,4-Dihydroxyphenyl | OH | H | H | 115.5 | less effective with 2-OH [2-hydroxy-2-(3,4-dihydroxyphenyl)ethanamine] |
| 11 | 3,4-Dihydroxyphenyl | OH | H | CH₃ | 135.0 | Small increase with R3 [2-hydroxy- 2-(3,4-dihydroxyphenyl)-N-methylethanamine] |
| 12 | 3,4-Dihydroxyphenyl | OH | CH₃ | H | 129.0 | small increase with R₂-CH₃ [1-hydroxy-1-(3,4-dihydroxyphenyl)-2-propanamine] |
| 13 | Phenyl | OH | CH₃ | CH₃ | 134.5 | no further increase with additional -CH₃ |
| 14 | Imidazole | (Ar only, no rest C - C chain) | | | 230.0 | at 0.1 mM |
| 15 | Imadazol-4-yl- | H | H | H | 150.0 | Histamine |
| 16 | Imadazol-4-yl- | H | COOH | H | 170.0 | Histidine; some increase in effectiveness |
| 17 | 5-Methylirnidazole-4-yl- | H | H | H | 130.5 | 5-methyl, less effective |

In another exemplary embodiment of the invention, the activator compounds may be imidazole compounds and their structural analogs, derivatives and salts, having the general structure: 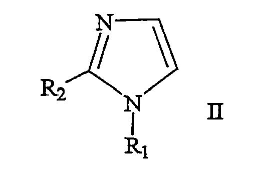 wherein R¹ and R² are independently H or lower alkyl, for example linear, branched or cyclic C₁-C₆ alkyl or C₁-C₄ alkyl. Methyl and ethyl are examples of lower alkyl groups that may be in position R¹. Methyl is an example of R².

In another exemplary embodiment of the invention, the activator compounds are linked di-imidazole compounds, derivatives and salts, having the general structure: 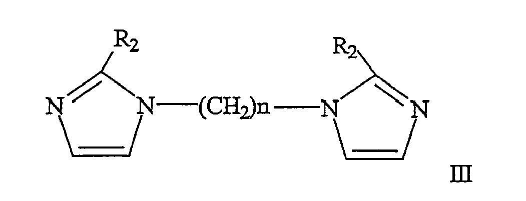 wherein *n* is 1 or 2 and R² is H or lower alkyl, for example linear, branched or cyclic C₁-C₆ alkyl or C₁-C₄ alkyl.

Different R groups may heighten the activator effect and associated cognitive enhancement. Such enhanced effects are readily determined by routine experimentation. Examples of imidazole compounds of the invention (structure II, compounds 18-21) and linked di-imidazoles of the invention (structure III, compounds 22-25) are shown in Tables 2 and 3, respectively. Triazoles and substituted triazoles may also be used as an alternative for imidazoles and substituted imidazoles in any of the general structures I, II and III.

**Table 2**

| **Effector** | **R₁** | **R₂** | **%Activity** |
|---|---|---|---|
| 18 | H | H | 190 |
| 19 | CH₃ | H | 194 |
| 20 | C₂H₅ | H | 203 |
| 21 | CH₃ | C₂H₅ | 247 |

**Table 3**

| **Effector** | ***N*** | **R₁** | **%Activity** |
|---|---|---|---|
| 22 | 1 | H | 140 [di(1-imidazolyl)methane] |
| 23 | 1 | CH₃ | 169 [di(2-methyl-1-imidazolyl)methane] |
| 24 | 2 | H | 154 [1,2-di(1-imidazolyl)ethane] |
| 25 | 2 | CH₃ | 131 [1,2-di(2-methyl-1-imidazolyl)ethanel |

Some of these compounds were tested on rats in learning and memory experiments. The results are graphed in Figures 4a and 4b. The activation of carbonic anhydrase is shown to be directly related to learning and memory effects in a mammal. Reduced activity inhibits learning and memory. Increased activity improves learning and memory in a linear proportional manner.

Compounds of the invention are set forth in the above referenced Tables 1, 2 and 3. Many of these compounds are already known and the methods for obtaining them are known to persons of ordinary skill. The compounds may be combined and may be administered in a pharmaceutically acceptable carrier, and packaged together with labeling indicating a cognitive effect.

The invention encompasses derivatives and analogs of these compounds which increase the potency of the carbonic anhydrase activating effect, increase the specificity to carbonic anhydrase as compared to other targets, reduce toxicity, improve stability in an oral dosage form, and/or enhance the ability of the compound to cross the blood brain barrier (pro-drugs). Derivatives are compounds formed by adding or removing side chains from the listed compounds. Analogs are structural variants of the compounds having enhanced similar physical and/or chemical properties with respect to the binding site of carbonic anhydrase. Derivatives and analogs according to the invention are those which are able to deliver the activator compounds of the invention to the brain of a subject.

The compounds of the present invention may provide neuronal carbonic anhydrase activity of at least about 110, 115, 125, 135, 150, 170, 180, 190, 200, 210, 220, 230, 240 and 250% that of alanine.

The effective dose for administration of the compounds is one that enhances carbonic anhydrase activity in cells of neuronal signaling pathways associated with learning particular tasks, attention, and memory. When the activator compounds are administered in effective doses according to the invention, they enhance carbonic anhydrase activity by either directly activating carbonic anhydrase or by inducing the calcium-signaling intracellular neuronal pathway to activate carbonic anhydrase. If a dose is too high, there is no beneficial learning effect and indeed the subject may demonstrate impaired learning. Thus, a large dose may overwhelm the neuronal pathways and a small dose may not achieve the desired enzyme activation and learning effect. The dosage must be adjusted to get the desired result.

Extrapolating from rat dosing, which is predictive of human dosing, effective doses of a phenylalanine (50 mM) or imidazole (0.5 M) agents for treating humans may include the equivalent of 0.1, 0.3, 1, 3 or 10 ml/kg body weight taken twice per day. A desirable dosing regimen includes administering the compound about 30 minutes prior to desired attentive cognition activity.

The chemical compositions useful in the present invention can be "converted" into pharmaceutical compositions by the dissolution in, and/or the addition of, appropriate, pharmaceutically acceptable carriers or diluents. Thus, the compositions may be formulated into solid, semi-solid, liquid, or gaseous preparations, such as tablets, capsules, powders, granules, ointments, solutions, suppositories, injectables, inhalants, and aerosols, using conventional means. Known methods are used to prevent release or absorption of the active ingredient or agent until it reaches the target cells or organ or to ensure time-release of the agent. A pharmaceutically acceptable form is one which does not inactivate or denature the active agent. In pharmaceutical dosage forms useful herein, the present compositions may be used alone or in appropriate association or combination with other pharmaceutically active compounds.

Accordingly, the pharmaceutical compositions of the present invention can be administered to any of a number of sites of a subject and thereby delivered via any of a number of routes to achieve the desired effect. Local or systemic delivery is accomplished by administering the pharmaceutical composition via injection, infusion or sintillation into a body part or body cavity, or by ingestion, inhalation, or insufflation of an aerosol. Preferred routes of administration include parenteral administration, which includes intramuscular, intracranial, intravenous, intraperitoneal, subcutaneous intradermal, or topical routes.

The present compositions can be provided in unit dosage form, wherein each dosage unit, e.g., a teaspoon, a tablet, a fixed volume of injectable solution, or a suppository, contains a predetermined amount of the composition, alone or in appropriate combination with other pharmaceutically active agents. The term "unit dosage form" refers to physically discrete units suitable for a human or animal subject, each unit containing, as stated above, a predetermined quantity of the present pharmaceutical composition or combination in an amount sufficient to produce the desired effect. Any pharmaceutically acceptable diluent or carrier may be used in a dosage unit, e.g., a liquid carrier such as a saline solution, a buffer solution, or other physiologically acceptable aqueous solution), or a vehicle. The specifications for the novel unit dosage forms of the present invention depend on the particular effect to be achieved and the particular pharmacodynamic properties of the pharmaceutical composition in the particular host.

An "effective amount" of a composition is an amount that produces the desired effect in a host, which effect can be monitored, using any end-point known to those skilled in the art. The methods described herein are not intended to be all-inclusive, and further methods known to those skilled in the art may be used in their place.

Furthermore, the amount of each active agent exemplified herein is intended to provide general guidance of the range of each component which may be utilized by the practitioner upon optimizing these methods for practice either *in vitro* or *in vivo.* Moreover, exemplified dose ranges do not preclude use of a higher or lower doses, as might be warranted in a particular application. For example, the actual dose and schedule may vary depending on (a) whether a composition is administered in combination with other pharmaceutical compositions, or (b) inter-individual differences in pharmacokinetics, drug disposition, and metabolism. Similarly, amounts may vary for in vitro applications. One skilled in the art can easily make any necessary adjustments in accordance with the necessities of the particular situation.

There are several isozymes of carbonic anhydrase. See Lindskog, "Structure and Function of Carbonic Anhydrase, "Pharmacol. Ther. Vol.74 (1), P1-20, 1997. The structure of the CAII binding site for acetazolamide and some other inhibitors is known. This knowledge allows rational design of derivatives and analogs of the compounds listed herein.

### EXAMPLE

### Introduction

CA1 pyramidal cells were recorded in rat hippocampal slices. In the presence of carbonic anhydrase activators, comicrostimulation of cholinergic inputs from stratum oriens and γ-aminobutyric acid (GABA)ergic inputs from stratum pyramidale at low intensities switched the hyperpolarizing GABA-mediated inhibitory postsynaptic potentials to depolarizing responses. In the absence of the activators, however, the same stimuli were insufficient to trigger the synaptic switch. This synaptic switch changed the function of the GABAergic synapses from excitation filter to amplifier and was prevented by carbonic anhydrase inhibitors, indicating a dependence on HCO₃. Intralateral ventricular administration of these same carbonic anhydrase activators caused the rats to exhibit superior learning of the Morris water maze task, suggesting that the GABAergic synaptic switch is critical for gating the synaptic plasticity that underlies spatial memory formation. Increased carbonic anhydrase activity also enhances perception, processing, and storing of temporally associated relevant signals and represents an important therapeutic target in learning and memory pharmacology.

### Materials and Methods

**Brain Slices**. Male Sprague-Dawley rats (150-180 g) were anesthetized with pentobarbital and decapitated. The hippocampal formation was removed and sliced (400 µm) with a McIllwain tissue chopper (Sun et al., 1999). Slices were maintained in an interface chamber (Medical systems Corp., Greenvale, NY) at 31°C with continuous perfusion of artificial cerebrospinal fluid. Artificial cerebrospinal fluid consisted of 125 mM NaCl, 3 mM KCl, 1.3 mM MgSO₄, 2.4 mM CaCl₂, 26 mM NaCHO₃, 1.25 mM NaH₂PO₄, and 10 mM C₆H₁₂O₆.

**Electrophysiology**. Intracellular recordings were obtained from CA1 pyramidal neurons using glass micropipette electrodes filled with 2 M potassium acetate (pH 7.25), with measured tip resistance in the range 70 to 120 MΩ. Cells that show obvious accommodation, an identifying characteristic of pyramidal cells, were used in the study. Labeling the recorded cells exhibiting this characteristic with dye has previously revealed that the recorded cells are indeed pyramidal cells (Sun et al., 1999). Signals were amplified, digitized, and stored using AxoClamp-2B amplifier and DigiData 1200 with the P-clamp data acquisition and analysis software (Axon Instruments, Foster City, CA). Stratum pyramidale, stratum radiatum, and/or stratum oriens were stimulated (about 200 µm from the recording electrode), using bipolar electrodes constructed of Teflon-insulated PtIr wire (25 µm in diameter, the approximate thickness of stratum pyramidale; FHC Inc., Bowdoinham, ME). Monophasic hyperpolarizing postsynaptic potentials (PSPs) were elicited by orthodromic single-pulse stimulation of interneurons in stratum pyramidale (Collin et al., 1995). In some experiments, a stimulating electrode (about 400 µm from the other stimulating electrodes when two stimulating electrodes were placed) was also placed in stratum oriens to activate cholinergic terminals and evoke acetylcholine release (Cole and Nicoll, 1984), or in stratum radiatum to evoke glutamatergic PSPs. Costimulation of stratum oriens and stratum pyramidale consisted of stimulation of stratum oriens with single pulses (20-60 µA and 50 µs, 1 Hz for 10 s) and stimulation of stratum pyramidale with four trains [10 pulses/train at control intensity (30-60 µA and 50 ms, 100 Hz), starting at the ninth stratum oriens stimulation] at a 0.5-s intertrain interval.

Drugs and Ligands. Bicuculline, acetazolamide (solubilized in dimethyl sulfoxide), kynurenic acid, imidazole, phenylalanine, and atropine were from Sigma (St. Louis, MO) and were solubilized in the noted concentrations and delivered to the slice chamber from an external reservoir. For intralateral ventricular injections of phenylalanine (50 mM), imidazole (0.5 M), and or acetazolamide (10 mM) in vivo, agents (2 µl/site/day) were bilaterally injected during training days about 30 min before the training, at a speed of 1 µl/min. The control rats received the same volume of saline.

**Spatial Maze Tasks.** Effects of increasing HCO₃ formation in vivo on spatial memory were evaluated in rats with the Morris water maze task. Male adult Wistar rats (200-250 g) were housed in a temperature-controlled (20-24°C) room for a week, allowed free access to food and water, and kept on a 12-h light/dark cycle. Rats were anesthetized with sodium pentobarbital (60 mg/kg i.p) and placed in a stereotactic apparatus (Kopf Instruments, Tujunga, CA). The core temperature of rats was monitored and kept constant (38.0 ± 0.5°C) with warming light and pad. Two stainless steel guide cannulas were placed with the tips positioned at the coordinates (anterior-posterior, 0.5 mm; lateral, 1.5 mm; horizontal, 3.2 mm), under aseptic conditions. At the end of surgery and under appropriate anesthesia, rats received (s.c.) banamine (1 mg/kg) and ketoprofen (5 mg/kg) in lactate/Ringer' solution. A 7-day recovery period was allowed before any further experimentation.

On the first day of experiments, all rats were randomly assigned to different groups (10 each) and swam for 2 min in a 1.5- (diameter) X 0.6-m (depth) pool (22 ± 1°C). On the following day, rats were trained in a two-trial per day task for four consecutive days. Each training trial lasted for up to 2 min, during which rats learned to escape from water by finding a hidden platform that was placed at a fixed location and submerged about 1 cm below the water surface. The navigation of the rats was tracked by a videocamera. The escape latency and the route of rats' swimming across the pool to the platform were recorded. The quadrant test (1 min) was performed after removing the platform, 24 h after the last training trial.

Statistical analyses were performed using the Student's *t* test for paired or unpaired data or ANOVA whenever appropriate. The values are expressed as means ± S.E.M., with *n* indicating the number of the cells or rats. All animals used in these experiments were treated under National Institutes of Health guidelines for the welfare of laboratory animals.

### Results

Microstimulation of stratum pyramidale with a single pulse elicited a hyperpolarizing inhibitory postsynaptic potential (IPSP; Fig. 1a). The IPSP was, mainly if not exclusively, from activation of the GABAergic inputs from the Basket interneurons, whose cell bodies and axons are restricted to stratum pyramidale. As described in previous publications (Sun et al., 1999, 2000), the IPSPs exhibited a reversal potential of about -78 mV. No detectable minor PSP components that exhibit a different reversal potential were observed. Bath application of kynurenic acid (500 µM, 20 min), a broad-spectrum competitive antagonist for both N-methyl-D-aspartate (NMDA) and non-NMDA receptors (Collingridge and Lester, 1989), effectively abolished EPSPs of CA1 pyramidal cells evoked by stimulation of the Schaffer collateral pathways (Sch; by 96.3 ± 4.1%, *n* = 6 from six different rats, *p* < 0.05). At this concentration, kynurenic acid did not increase the IPSP amplitudes (-8.2 ± 0.6 mV prekynurenic acid versus 28.3 ± 0.7 mV during the application; *n* = 7 from seven different rats, *p* > 0.05; Fig. 1a), suggesting that the single-pulse stratum pyramidale micro-stimulation did not evoke a significant glutamatergic EPSP component. The IPSPs, however, were blocked by bicuculline, the selective GABA_{A} receptor antagonist (by 97.9 ± 4.4% on average, *n* = 6 from six different rats, *p* < 0.05; 1 µM, 30-min perfusion; Fig. 1b), indicating that the IPSPs were predominantly mediated by activation of the GABA_{A} receptors and were therefore referred to as Basket interneuron-CAl responses.

Single-pulse stimulation of stratum oriens (1 Hz, 10 s) coincident with trains of stimulation of stratum pyramidale produced a small but lasting decrease in the IPSP amplitudes (Fig. 1, d and f). For instance, at 40 min after the costimulation, the peak IPSPs were -4.9 ± 0.7 mV, significantly smaller than -7.4 ± 0.9 mV before the associated stimulation *(n* = 8 from seven different rats, *p* < 0.05; paired *t* test). Two carbonic anhydrase activators, imidazole (100 µM, 20 min; Parkes and Coleman, 1989) or phenylalanine (100 µM, 20 min; Clare and Supuran, 1994), were applied. In the presence of phenylalanine, the peak IPSPs in response to single-pulse stimulation of stratum pyramidale were slightly but significantly reduced (Fig. 1c; to -4.5 ± 0.8 mV in the presence of phenylalanine from prephenylalanine IPSPs of -7.6 ± 1.2 mV; *n* = 7 from seven different rats, *p* < 0.05). In the presence of the carbonic anhydrase activator, the same intensities of costimulation of stratum pyramidale and stratum oriens produced a lasting reversal of the IPSPs to EP-SPs, observed when the membrane potentials were maintained at their control levels (Fig. 1, d and e). Thus, 40 min after the costimulation (under *Materials and Methods)* and in the presence of phenylalanine, the peak PSPs were 6.4± 1.1 mV, significantly different *(n* = 8 from eight different rats, *p* < 0.05) from their prephenylalanine values (-7.2 ± 1.2 mV) or from those in the presence of phenylalanine but before the costimulation (Fig. 1d). In the presence of imidazole, similar effects on the IPSPs (-5.3 ± 0.7 mV in the presence of imidazole versus preimidazole of-7.8 ± 0.6 mV; *n* = 7 from seven different rats, *p* < 0.05) and effects of the costimulation (peak PSPs: 4.2 ± 0.6 mV, in the presence of imidazole and 40 min after the costimulation versus preimidazole values of-7.5 ± 0.7 mV; *n* = 6 from six different rats, *p* < 0.05) were observed, although in general, less potent. Thus, the results with imidazole were not illustrated in detail.

Both the reducing effect of carbonic anhydrase activators on the IPSPs and the synaptic switching effect with costimulation of the cholinergic and GABAergic inputs depend on activity of the carbonic anhydrase. For instance, in the presence of acetazolamide (10 µM, 20 min), a blocker of carbonic anhydrase and thus the synthesis of HCO₃ (Staley et al., 1995), phenylalanine did not significantly reduce the peak IPSPs (-7.7 ± 0.9 mV in the presence of phenylalanine versus prephenylalanine peak IPSPs of-7.9 ±1.1 mV, *n* = 6 from six different rats, *p* > 0.05). Nor did imidazole, in the presence of acetazolamide, significantly change the size of the IPSPs (-7.5 ± 1.0 mV in the presence of imidazole versus preimidazole peak IPSPs of -7.4 ± 0.8 mV, *n* = 5 from five different rats, *p* > 0.05). The same intensities of costimulation did not induce the synaptic switch (Fig. 1, d and g) in the presence of acetazolamide and phenylalanine or imidazole. Thus, in the presence of acetazolamide and phenylalanine, these IPSPs were not significantly altered by the costratum oriens stratum pyramidale stimulation (-7.8 ± 1.3 mV, 40 min after compared with -7.6 ± 0.9 mV control value, *n* = 8 from eight different rats, *p* > 0.05). Furthermore, the co-stimulation did not significantly alter the IPSPs in the presence of acetazolamide and imidazole (-7.7 ± 1.1 mV, 40 min after compared with -7.5 ± 0.8 mV control value, *n* = 6 from six different rats, *p* > 0.05).

The influence of the GABAergic synaptic switch on the signal passage through the CA1 cells was evaluated when the glutamatergic Sch inputs were costimulated. In eight cells, single-pulse stratum pyramidale stimulation evoked an IPSP (Fig. 2a). Excitatory Sch input was stimulated at intensities 30% above the action potential threshold (100% of 20 trials) of the recorded cells (Fig. 2b). Costimulation of the GABAergic inputs and Sch blocked (100% of 20 trials; *n* = 10 from eight different rats, *p* > 0.05) the effects of excitatory Sch input, stimulated at above-action-potential-threshold intensities (Fig. 2c) in all eight cells tested. The effective signal-filtering period in each single-pulse-evoked inhibitory response was ≥100 ms, during which no action potential (0% of 20 trials) was evoked by Sch stimulation at the above-threshold intensity. After the synaptic switch (Fig. 2d) induced by costimulation of the GABAergic and cholinergic inputs in the presence of phenylalanine, below-threshold Sch stimulation, which by itself did not evoke action potentials (0% of 20 trials; Fig. 2e), became sufficient to evoke action potentials (100% of 20 trials; *n=* 8 from eight different rats, *p* < 0.05) when delivered during the period of $100 ms of single-pulse stimulation of the GABAergic input (Fig. 2f; *n* = 8 from eight different rats). Multiple spikes were evoked when the Basket interneurons-CA1 PSP was costimulated with above-thresh-old Sch stimulation after inducing the synaptic switch (data not shown). Thus, after the synaptic switch, activity of the GABAergic interneurons amplified excitatory Sch inputs. Therefore, weak signals are amplified after synaptic switch o trigger action potentials, while strong excitatory signals cannot successfully pass through the network under associated inhibition.

The effects of carbonic anhydrase activators were tested on spatial learning in rats, using the hidden-platform water maze. As shown in Fig. 3a, the latency to escape to the platform in all three groups of rats decreased following the training sessions. Statistical analysis revealed significant effects of groups (F_{2,27} = 9.192, *p* < 0.001), trials (F_{7,218} = 7.83, *p* < 0.001), and groups X session of trials (F_{14,218} =3.70, *p* < 0.001), indicating that spatial learning in rats injected with phenylalanine (phenylalanine rats) was faster than in rats injected with saline (control rats). Moreover, a post hoc analysis reveals a significant difference from the second to sixth trials *(p* < 0.05), confirming better learning in phenylalanine rats. In fact, the escape latency of the phenylalanine rats reached a plateau on the fifth trial. Three additional trials were needed for the control rats to show the same escape latency as the phenylalanine rats (Fig. 3a). Quadrant tests 24 h after the last training trial revealed that the control rats (F_{3,36} = 159.9, *p* < 0.0001; ANOVA and Newman-Keuls post hoc test), and the phenylalanine rats (F_{3,36} = 201.2, *p* < 0.0001) spent more time searching in the target quadrant (quadrant 4) where the platform was previously placed and had been removed. However, in comparison with control rats, phenylalanine rats exhibited a clearly greater preference for the target quadrant (by 24.8 ± 1.8%, *p* < 0.05; unpaired *t* test) (Fig. 3, d and e). The target quadrant ratios, target/average of the nontarget quadrants, between the pheynlalanine and the control rats were significantly different *(p* < 0.001; Fig. 3b). Similarly, rats injected with imidazole (imidazole rats) also showed a faster learning and a significant shorter escape latency from the third to sixth trials *(p* < 0.05) than the control animals. Quadrant tests revealed that imidazole rats had a greater preference for the target quadrant (by 15.1 ± 1.6%,*p* < 0.05) than the control rats. Thus, the rats injected with the carbonic anhydrase activators performed better than their controls in this spatial memory retention task. The average swim speeds for all eight trials, however, did not differ between all the groups (Fig. 3c; *p* > 0.05), including the imidazole and acetazol-amide/imidazole groups (data not shown), indicating that the carbonic anhydrase activators and inhibitor did not grossly affect their sensory or locomotor activities. During the experimental periods, no rats showed any apparent sign of discomfort or abnormal behaviors such as hypo- or hyperactivity.

The effects of carbonic anhydrase activators on spatial learning were sensitive to carbonic anhydrase inhibitors. Bilateral intraventricular injections of acetazolamide not only eliminated the effects of the carbonic anhydrase activators on the learning but also produced memory impairment (Fig. 3a). The acetazolamide/phenylalanine group showed a strikingly smaller reduction (F_{1,18} = 40.38, *p* < 0.0001) in escape latency during training trials than the control group did. Quadrant tests revealed that the acetazolamide/phenylalanine rats showed no significantly different preference for a particular quadrant (F_{3,36} = 1.43, *p* > 0.05; Fig. 3f) and a significantly different (*p* < 0.001) target quadrant ratio from those of the phenylalanine and the control rats (Fig. 3b). Identical results were also observed in rats injected with acetazolamide and imidazole (data not shown).

According to the invention, enhancement of the GABAergic synaptic switch in controlling signal processing in the hippocampal network can be achieved through the use of carbonic anhydrase activators, and these carbonic anhydrase activators increase efficacy of temporally associated activity of the cholinergic and GABAergic inputs in switching the hyperpolarizing GABAergic IPSPs to excitatory PSPs. The synaptic switch can be induced by associative postsynaptic stimulation (Collin et al., 1995), activation of the calexcitin signal cascade, or costimulation of the cholinergic and GABAergic inputs at greater intensities and more prolonged periods of stimulation (Sun et al., 2001 a). The results shown above indicate that the presence of the enzyme activators facilitates induction of the synaptic switch so that weaker and fewer trains of costimulation were required. Thus, administration of carbonic anhydrase activators may additively or synergistically augment a naturally occurring activation of carbonic anhydrase that occurs in neurons of pathways associated with attentive cognition.

Two enzyme activators from different classes of compounds, which have different spectra of biological actions, were used in the study, yielding similar results. They were administered directly into the brain to avoid the limitation of accumulation in the brain by the blood-brain barrier. Competitive transport and rapid peripheral hydroxylation are known to limit the phenylalanine concentration in the brain of systemically administered phenylalanine-containing substances (such as aspartame, whose metabolites include 5-benzyl-3,6-dioxo-2-piperazineacetic acid, phenylalanyl aspartic acid, asparaginyl-phenylalanine, phenylalanine methyl ester, phenylalanine, aspartic acid, methanol, and formate). These effects limit phenylalanine's access to the brain and possibly its behavioral impact In addition to activation of carbonic anhydrase, high concentrations of phenylalanine in the brain might facilitate the synthesis of catecholamines and catecholaminergic transmission.

Imidazole-like structures, on the other hand, may react with many biologically active molecules, including monoamine oxidase, histamine H₂ receptors, angiotensin II type 1 receptors, ethanol binding sites in GABA receptor channel complex, GABA_{c} receptors, the nicotinic-cholinergic receptor channel complex, the prosthetic heme group of the nitric-oxide synthase, some K_{ATP} channels, and imidazole binding sites. The biological consequences and specificity of an increased brain imidazole concentration, therefore, still remain to be clarified. Thus, these results do not rule out a possible conttibution of synaptic/signal interaction in other brain regions or an action of the substances and their metabolites at the α-adrenoceptors, dopaminergic receptors, and/or histaminergic receptors to the enhancement of spatial learning and memory. The common denominator of the two carbonic anhydrase activators, the action on carbonic anhydrase, however, is the likely underlying mechanism for the observed effects. The critical role of carbonic anhydrase activation in the observed effects of carbonic anhydrase activators was further directly demonstrated by the effectiveness of acetazolamide, a carbonic anhydrase inhibitor, in blocking the synaptic switch. Acetazolamide has been shown to be able to reduce or eliminate flux of HCO₃ in hippocampal pyramidal neurons underlying a depolarizing PSP (Staley et al., 1995). Activity of carbonic anhydrase in the CA1 pyramidal cells is essential since intracellular application of benzolamide, a membrane-impermeant carbonic anhydrase inhibitor, was previously found to effectively block the GABAergic synaptic switch (Sun et al., 1999).

Carbonic anhydrase is a highly efficient enzyme. If its activity is crucial for coding and storing learned information, one would expect the existence of cellular mechanisms to control activity of the enzyme. There are indications that intracellular Ca²⁺ release increases HCO₃ conduction through the GABA_{A} receptor-mediated IPSPs and that the effect is sensitive to carbonic anhydrase inhibition (Sun et al., 2000). Membrane association is another efficient mechanism to activate carbonic anhydrase (Parkes and Coleman, 1989). Translocation and membrane association of the cytosol carbonic anhydrase may participate in memory acquisition and/or consolidation. The inventive method permits activation of neuronal carbonic anhydrase by any or all such mechanisms. The involvement of carbonic anhydrase in cognitive functions is consistent with the evidence (Meier-Ruge et al., 1984) of a significantly diminished activity of the enzyme in Alzheimer's disease than in age-matched controls and with increasing age.

The present results demonstrate that the switched synaptic responses provide a postsynaptic mechanism to direct or gate signal flow through the hippocampal network. The GABAergic interneurons, especially the Basket interneurons, whose cell bodies and axons are restricted in the cell layer, are known to innervate the perisomatic region of the pyramidal cells. Thus, bursting activity from the interneurons in the absence of synaptic switch inhibits the pyramidal cells, powerfully blocking excitatory signal transfer through the hippocampal circuit. An associated activation of the cholinergic and GABAergic inputs can trigger the synaptic switch, especially when the carbonic anhydrase is activated. After the synaptic switch, however, the same type of GABAergic activity amplifies excitatory signal. The mechanism thus differentiates responses according to the nature and temporal association of relevant signals and the neural activity states, a phenomena that may underlie synaptic plasticity in learning and memory (Liu and Cull-Candy, 2000; Shulz et al., 2000). The synaptic switch mechanism enables the network to perform signal processing and gate information flow and direction accordingly.

Thus according to the invention, altering the neural activity states that learning depends on via carbonic anhydrase activity represents an effective therapeutic strategy to achieve memory therapy. Agents that activate carbonic anhydrase according to the invention have clinical value for enhanced memory and for the treatment of spatial memory decline. Phenylalanine may be used in the majority of individuals who do not have genetic lack of phenylalanine hydroxylase, and more potent and selective nonphenylalanine activators (such as imidazole-and histamine-derivatives) can help individuals with hydroxylase dysfunction.

The embodiments illustrated and discussed in this specification are intended only to teach those skilled in the art the best way known to the inventors to make and use the invention. Nothing in this specification should be considered as limiting the scope of the present invention. The above-described embodiments of the invention may be modified or varied, and elements added or omitted, without departing from the invention, as appreciated by those skilled in the art in light of the above teachings. It is therefore to be understood that, within the scope of the claims and their equivalents, the invention may be practiced otherwise than as specifically described.

### REFERENCES

The following documents are incorporated herein by reference:
1. Alkon DL, Nelson TJ, Zhao WQ and Cavallaro S (1998) Time domains of neuronal Ca2+ signaling and associative memory: steps through a calexcitin, ryanodine receptor, K+ channel cascade. Trends Neurosci 21:529-537.
2. Alkon DL, Sanchez-Andres JV, Ito E, Oka K, Yoshioka T and Collin C (1992) Long-term transformation of an inhibitory into an excitatory GABAergic synaptic response. Proc Natl Acad Sci USA 85:11862-11866.
3. Banks M, White JA and Pearce RA (2000) Interactions between distinct GABAA circuits in hippocampus. Neuron 25:449-457.
4. Bartus RT, Reginald L, Dean RL, Beer B and Lippa AS (1982) The cholinergic hypothesis of geriatric memory dysfunction. Science (Wash DC) 217:408-414.
5. Buhl EH, Cobb SR, Halasy K and Somogyi P (1995) Properties of unitary IPSPs evoked by anatomically identified basket cells in the rat hippocampus. Eur J Neurosci 7:1989-2004.
6. Burg M, Heinemann U and Schmitz D (1998) Neuroactive steroids induce GABAA receptor-mediated depolarizing postsynaptic potentials in hippocampal CAI pyra-midal cells of rats. Eur J Neurosci 10:2880-2896.
7. Christie BR, Kerr DS and Abraham WC (1994) Evidence for common expression mechanisms underlying heterosynaptic and associative long-term depression in the dentate gyrus. Hippocampus 4:127-135.
8. Clare BW and Supuran CT (1994) Carbonic anhydrase activators. 3: Structure-activity correlations for a series of isozyme II activators. J Pharmacol Sci 83:768-773.
9. Cobb SR, Buhl EH, Halasy K, Paulsen O and Somogyi P (1995) Synchronization of neuronal activity in hippocampus by individual GABAergic interneurons. Nature (Lond) 378:75-78.
10. Cole AE and Nicoll RA (1984) Characterization of a slow cholinergic post-synaptic potential recorded in vitro from rat hippocampal pyramidal cells. J Physiol (Lond) 352:173-188.
11. Collin C, Devane WA, Dahl D, Lee C-J, Axelrod J and Alkon DL (1995) Long-term synaptic transmission of hippocampal CA1 γ-aminobutyric acid synapses and the effect of anandamide. Proc Natl Acad Sci USA 92:10167-10171.
12. Collingridge GL and Lester RA (1989) Excitatory amino acid receptors in the vertebrate central nervous system. Pharmacol Rev 40:143-209.
13. Cooper JD and Sofroniew MV (1996) Increased vulnerability of septal cholinergic neurons to partial loss of target neurons in aged rats. Neuroscience 75:29-35.
14. Dickinson-Anson H, Aubert I, Gage FH and Fisher LJ (1998) Hippocampal grafts of acetylcholine-producing cells are sufficient to improve behavioral performance following a unilateral fimbria-fornix lesion. Neuroscience 84:771-781.
15. Kaila K, Lamsa K, Smimov S, Taira T and Voipio J (1997) Long-lasting GABA-mediated depolarization evoked by high-frequency stimulation in pyramidal neurons of rat hippocampal slice is attributable to a network-driven, bicarbonate-dependent K+ transient. J Neurosci 17:7662-7672.
16. Kornhauser JM and Greenberg ME (1997) A kinase to remember: dual roles for MAP kinase in long-term memory. Neuron 18:839-842.
17. Leinekugel X, Khalilov I, McLean H, Caillard O, Gaiarsa JL, Ben-Ari Y and Khazi-pov R (1999) GABA is the principal fast-acting excitatory transmitter in the neonatal brain. Adv Neurol 79:189-201.
18. Lindskog, "Structure and Function of Carbonic Anhydrase, "Pharmacol. Ther. Vol.74 (1), P1-20, 1997.
19. Liu S-QJ and Cull-Candy SG (2000) Synaptic activity at calcium-permeable AMPA receptors induces a switch in receptor subtype. Nature (Lond) 405:454-458.
20. Meier-Ruge W, IwangoffP and Reichlmeier K (1984) Neurochemical enzyme changes in Alzheimer's and Pick's disease. Arch Gerontol Geriatr 3:161-165.
21. Ohno M, Kobayashi M, Kishi A and Watanabe S (1997) Working memory failure by combined blockade of muscarinic and β-adrenergic transmission in the rat hip-pocampus. Neuroreport 8:1571-1575.
22. Parkes JL and Coleman PS (1989) Enhancement of carbonic anhydrase activity by erythrocyte membranes. Arch Biochem Biophys 275:459-468.
23. Pasternack M, Voipio J and Kaila K (1993) Intracellular carbonic anhydrase activity and its role in GABA-induced acidosis in isolated rat hippocampal pyramidal neurons. Acta Physiol Scand 148:229-231.
24. Paulsen O and Moser EI (1998) A model of hippocampal memory encoding and retrieval: GABAergic control of synaptic plasticity. Trends Neurosci 21:273-278.
25. Perry E, Walker M, Grace J and Perry R (1999) Acetylcholine in mind: a neurotransmitter correlate of consciousness? Trends Neurosci 22:273-280.
26. Shulz DE, Sosnik R, Ego V, Haidarliu S and Ahissar E (2000) A neuronal analogue of state-dependent learning. Nature (Lond) 403:549-553.
27. Sone M, Sei H, Morita Y, Ogura T and Sone S (1998) The effects of acetazolamide on arterial pressure variability during REM sleep in the rat. Physiol Behav 63:213-218.
28. Staley KJ, Soldo BL and Proctor WR (1995) Ionic mechanisms of neuronal excitation by inhibitory GABAA receptors. Science (Wash DC) 269:977-981.
29. Steckler T, Sahgal A, Aggleton JP and Drinkenburg WH (1998) Recognition memory in rats. III. Neurochemcial substrates. Prog Neurobiol 54:333-348.
30. Sun MK, Alkon DL., "Pharmacological Enhancement of Synaptic Efficacy, Spatial Learning, and Memory Through Carbonic anhydrase Activation in Rats," JPET 297:961-967 (2001).
31. Sun M-K, Zhao W-Q, Neslon TJ and Alkon DL (2001b) Theta rhythm of CA1 hippocampal neuron activity: gated by GABAergic synaptic depolarization. J Neu-rophysiology 85:269-279.
32. Sun, M.K., Dennis Dahl and Daniel L. Alkon, "Heterosynaptic Transformation of GABAergic Gating in the Hippocampus and Effects of Carbonic Anhydrase Inhibition," JPET 2001;297(3): 811-817, March 2001
33. Sun, M.K., Thomas J. Nelson, and Daniel L. Alkon, "Functional switching of GABAergic synapses by ryanodine receptor activation," Proc. Natl. Acad. Sci. USA vol. 97, pp. 12300-12305 (October 24, 2000).
34. Sun, M.K., Thomas J. Nelson, Hui Xu, and Daniel L. Alkon, "Calexcitin transformation of GABAergic synapses: From excitation filter to amplifier," Proc. Natl. Acad. Sci. USA vol. 96, pp. 7023-7028 (June 8, 1999).
35. Taira T, Lamsa K and Kaila K (1997) Posttetanic excitation mediated by GABAA receptors in rat CA1 pyramidal neurons. J Neurophysiol 77:2213-2218.
36. Tang YP, Shimizu E, Dube GR, Rampon C, Kerchner GA, Zhuo M, Liu G and Tsien JZ (1999) Genetic enhancement of learning and memory in mice. Nature (Lond) 401:63-69.
37. Winkler J, Suhr ST, Gage FH, Thal LJ and Fisher LJ (1995) Essential role of neocortical acetylcholine in spatial memory. Nature (Lond) 375:484-487.
38. Winson J (1978) Loss of hippocampal theta rhythm results in spatial memory deficit in the rat. Science (Wash DC) 201:160-163.
39. Wong RK and Watkins DJ (1982) Cellular factors influencing GABA response in hippocampal pyramidal cells. J Neurophysiol 48:938-951.
40. Wu M, Shanabrough M, Leranth C and Alreja M (2000) Cholinergic excitation of septohippocampal GABA but not cholinergic neurons: implications for learning and memory. J Neurosci 20:3900-3908.
41. Xiang Z, Huguenard JR and Prince DA (1998) Cholinergic switching within neocortical inhibitory networks. Science (Wash DC) 281:985-988.

In the following clauses, preferred embodiments of the invention are described:
1. A method comprising administering to the brain of a subject in need of improved attentive cognition a carbonic anhydrase activator compound in a dose effective to improve attentive cognition, the carbonic anhydrase activator compound being selected from the group:
   (1) structure I 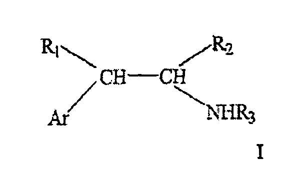 wherein R¹ is H or OH; R² and R³ are independently H, COOH or lower alkyl, for example linear, branched or cyclic C₁-C₆ alkyl or C₁-C₄ alkyl; and Ar is phenyl, imidazolyl or phenyl or imidazolyl substituted with one or more halo, hydroxy, amino or lower alkyl groups for example linear, branched or cyclic C₁-C₆ alkyl group or C₁-C₄ alkyl group;
   (2) structure II: 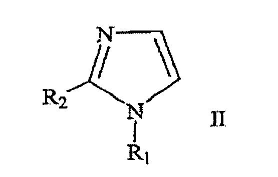 wherein R¹ and R² are independently H or lower alkyl, for example linear, branched or cyclic C₁-C₆ alkyl or C₁-C₄ alkyl;
   (3) structure III: 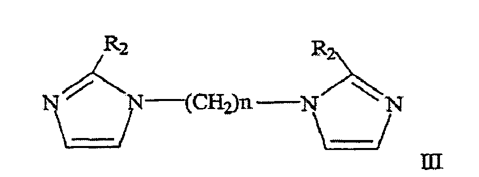
   wherein *n* is 1 or 2 and R² is H or lower alkyl, for example linear, branched or cyclic C₁-C₆ alkyl or C₁-C₄ alkyl; and salts thereof.
2. The method of clause 1, wherein the compound potentiates intraneuronal carbonic anhydrase activity.
3. The method of clause 1, wherein the activator has structure I and wherein R¹ is H or OH; R² is H, CH₃ or COOH; R³ is H or CH₃; and Ar is H, phenyl, 4-hydroxyphenyl, 4-fluorophenyl, 4-aminophenyl, 3-amino-4-hydroxyphenyl, 3,4-dihydroxyphenyl, imidazole, imadazol-4-yl-, or 5-methylimidazole-4-yl-.
4. The method of clause 1, wherein the activator has structure II and wherein R¹ is H, methyl or ethyl; and R² is H or methyl.
5. The method of clause 1, wherein the activator is structure III and wherein *n* is 1 or 2; and R² is H or methyl.
6. The method of clause 1, wherein the activator is selected from the group consisting of imidazole, alanine, phenylalanine, substituted ethylamine, phenethylamine, histamine, histidine, linked di-imidazole, triazole, and salts thereof.
7. The method of clause 1, wherein the carbonic anhydrase activator is administered as a pharmaceutical composition or in a pharmaceutically acceptable carrier.
8. The method of clause 1, wherein the patient has a neurodegenerative disorder.
9. The method of clause 1, wherein the method enhances cognitive ability, attention, learning, and/or memory in individuals without a neurological disorder.
10. The method of clause 1, wherein the method facilitates establishment of a theta rhythm via bicarbonate-mediated GABAergic depolarization.
11. The method of clause 10 wherein the method improves memory formation, learning, spatial memory, and/or attention.
12. The method of clause 10, wherein the method intervenes in the intracellular signaling cascade responsible for theta rhythm, the intervention comprising modulating HCO₃ conductance by directly altering intraneuronal carbonic anhydrase activity.
13. The method of clause 12, wherein the intervention modulates the HCO₃ current relative to the CI- and K⁺ currents.
14. The method of clause 1, wherein the method improves attentive cognition in a subject with Alzheimer's disease, stroke, hypoxia, and/or ischemia.
15. The method of clause 1, wherein the compound is one that provides carbonic anhydrase activity at least about 150% that of alanine in vitro.
16. The method of clause 1, wherein compound is one that provides carbonic anhydrase activity at least about 200% that of alanine in vitro.
17. The method of clause 1, wherein the compound is one that provides carbonic anhydrase activity at least about 250% that of alanine in vitro.
18. The method of clause 1, wherein the compound is administered to the brain by administering to the patient a pro-drug of an activator compound of clause 1, and allowing the prodrug to metabolize to the activator compound.
19. An article of manufacture comprising a pharmaceutical composition comprising an activator compound packaged together with labeling indicating use for improving attentive cognition, the activator compound being effective to enhance brain carbonic anhydrase activity and selected from
   (1) structure I 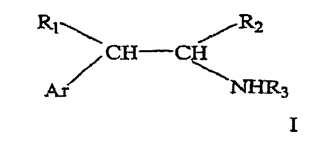 wherein R¹ is H or OH; R² and R³ are independently H, COOH or lower alkyl, for example linear, branched or cyclic C₁-C₆ alkyl or C₁-C₄ alkyl; and Ar is phenyl, imidazolyl or phenyl or imidazolyl substituted with one or more halo, hydroxy, amino or lower alkyl groups for example linear, branched or cyclic C₁-C₆ alkyl group or C₁-C₄ alkyl group;
   (2) structure II: 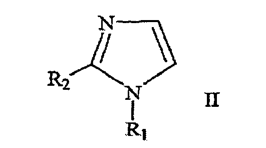 wherein R¹ and R² are independently H or lower alkyl, for example linear, branched or cyclic C₁-C₆ alkyl or C₁-C₄ alkyl;
   (3) structure III: 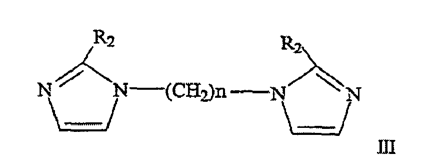
wherein *n* is 1 or 2 and R² is H or lower alkyl, for example linear, branched or cyclic C₁-C₆ alkyl or C₁-C₄ alkyl; and salts thereof.

## Claims

1. A carbonic anhydrase activator compound for improving attentive cognition in a dose effective to improve attentive cognition when administered to the brain of a subject in need of improved attentive cognition, the carbonic anhydrase activator compound being defined by structure I: 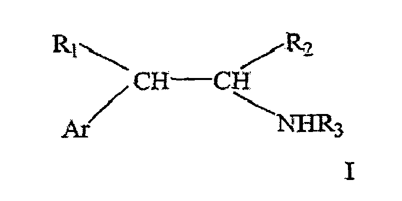 wherein R¹ is H or OH; R² and R³ are independently H, COOH or lower alkyl, for example linear, branched or cyclic C₁-C₆ alkyl or C₁-C₄ alkyl; and Ar is phenyl, imidazolyl or phenyl or imidazolyl substituted with one or more halo, hydroxy, amino or lower alkyl groups for example linear, branched or cyclic C₁-C₆ alkyl group or C₁-C₄ alkyl group.

2. The carbonic anhydrase activator compound of claim 1, wherein the compound potentiates intraneuronal carbonic anhydrase activity.

3. The carbonic anhydrase activator compound of claim 1, wherein the activator has structure I and wherein R¹ is H or OH; R² is H, CH₃ or COOH; R³ is H or CH₃; and Ar is H, phenyl, 4-hydroxyphenyl, 4-fluorophenyl, 4-aminophenyl, 3-amino-4-hydroxyphenyl, 3,4-dihydroxyphenyl, imidazole, imadazol-4-yl-, or 5-methylimidazole-4-yl-.

4. The carbonic anhydrase activator compound of claim 1, wherein the activator is selected from the group consisting of alanine, phenylalanine, substituted ethylamine, phenethylamine, histamine, histidine and salts thereof.

5. The carbonic anhydrase activator compound of claim 1, wherein the carbonic anhydrase activator is to be administered as a pharmaceutical composition or in a pharmaceutically acceptable carrier.

6. The carbonic anhydrase activator compound of claim 1, wherein the patient has a neurodegenerative disorder.

7. The carbonic anhydrase activator compound of claim 1, wherein the compound enhances cognitive ability, attention, learning, and/or memory in individuals without a neurological disorder.

8. The carbonic anhydrase activator compound of claim 1, wherein the compound facilitates establishement of a theta rhythm via bicarbonate-mediated GABAergic depolarization.

9. The carbonic anhydrase activator compound of claim 8, wherein the compound improves memory formation, learning, spatial memory, and/or attention.

10. The carbonic anhydrase activator compound of claim 8, wherein the compound intervenes in the intracellular signaling cascade responsible for the theta rhythm, the intervention comprising modulating HCO₃ conductance by directly altering intraneuronal carbonic anhydrase activity.

11. The carbonic anhydrase activator compound of claim 10, wherein the intervention modulates the HCO₃⁻ current relative to the Cl⁻ and K⁺ currents.

12. The carbonic anhydrase activator compound of claim 1, wherein the compound provides attentive cognition in a subject with Alzheimer's disease, stroke, hypoxia, and/or ischemia.

13. The carbonic anhydrase activator compound of claim 1, wherein the compound is one that provides carbonic anhydrase activity at least 150% that of alanine in vitro.

14. The carbonic anhydrase activator compound of claim 1, wherein the compound is one that provides carbonic anhydrase activity at least 200% that of alanine in vitro.

15. The carbonic anhydrase activator compound of claim 1, wherein the compound is one that provides carbonic anhydrase activity at least 250% that of alanine in vitro.

16. The carbonic anhydrase activator compound of claim 1, wherein the compound is administered to the brain by administering to the patient a prodrug of an activator compound of claim 1, and allowing the prodrug to metabolize to the activator compound.

17. An article of manufacture comprising a pharmaceutical composition comprising carbonic anhydrase activator compound packaged together with labeling indicating use of improving attentive cognition, the activator compound being effective to enhance brain carbonic anhydrase activity and being defined according to claim 1.
